(19) Europäisches Patentamt
European Patent Office
Office européen des brevets

(11) **EP 3 036 316 B1**

(12) **FASCICULE DE BREVET EUROPEEN**

(45) Date de publication et mention
de la délivrance du brevet:
**30.09.2020 Bulletin 2020/40**

(51) Int Cl.:
***C12M 1/00*** *(2006.01)*     ***C12P 21/00*** *(2006.01)*

(21) Numéro de dépôt: **14789311.9**

(86) Numéro de dépôt international:
**PCT/FR2014/052113**

(22) Date de dépôt: **22.08.2014**

(87) Numéro de publication internationale:
**WO 2015/025111 (26.02.2015 Gazette 2015/08)**

(54) **PROCEDE DE PRODUCTION INDUSTRIELLE DE FARINE DE BIOMASSE DE MICROALGUES RICHES EN LIPIDES SANS "OFF-NOTES" PAR CONTROLE DE LA DISPONIBILITE EN OXYGENE**

VERFAHREN ZUR INDUSTRIELLEN HERSTELLUNG VON MEHL AUS FETTREICHER MIKROALGENBIOMASSE OHNE OFF-NOTES DURCH STEUERUNG DER SAUERSTOFFVERFÜGBARKEIT

METHOD FOR THE INDUSTRIAL PRODUCTION OF FLOUR FROM LIPID-RICH MICROALGA BIOMASS WITH NO "OFF-NOTES" BY CONTROLLING THE OXYGEN AVAILABILITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.08.2013 FR 1358144**
**05.09.2013 FR 1358521**
**22.11.2013 FR 1361520**

(43) Date de publication de la demande:
**29.06.2016 Bulletin 2016/26**

(73) Titulaire: **Corbion Biotech, Inc.**
**South San Francisco, CA 94080 (US)**

(72) Inventeurs:
• **DELAROCHE, Sylvain**
**F-62219 Longuenesse (FR)**
• **LE RUYET, Marie**
**F-59000 Lille (FR)**
• **SEGUEILHA, Laurent**
**F-59520 Marquette Lez Lille (FR)**
• **JEROSCH, Heike**
**F-59940 Estaires (FR)**
• **DRUON, Amandine**
**F-59000 Lille (FR)**

(74) Mandataire: **Cabinet Becker et Associés**
**25, rue Louis le Grand**
**75002 Paris (FR)**

(56) Documents cités:
WO-A1-91/18108     WO-A2-2012/063137
CN-A- 102 618 431

• **SOO SUH: "Photobioreactor Engineering: Design and Performance", BIOTECHNOLOGY AND BIOPRESS ENGINEERING, vol. 8, 1 janvier 2003 (2003-01-01), pages 313-321, XP055105200,**
• **PETER HARMS ET AL: "Bioprocess monitoring", CURRENT OPINION IN BIOTECHNOLOGY, vol. 13, no. 2, 1 avril 2002 (2002-04-01), pages 124-127, XP055127989, ISSN: 0958-1669, DOI: 10.1016/S0958-1669(02)00295-1**
• **YEN-HUI CHEN ET AL: "Fed-batch fermentation and supercritical fluid extraction of heterotrophic microalgal Chlorella protothecoides lipids", BIORESOURCE TECHNOLOGY, vol. 114, 1 juin 2012 (2012-06-01), pages 512-517, XP055055165, ISSN: 0960-8524, DOI: 10.1016/j.biortech.2012.03.026**
• **XU H ET AL: "High quality biodiesel production from a microalga Chlorella protothecoides by heterotrophic growth in fermenters", JOURNAL OF BIOTECHNOLOGY, ELSEVIER SCIENCE PUBLISHERS, AMSTERDAM, NL, vol. 126, no. 4, 1 décembre 2006 (2006-12-01), pages 499-507, XP024956582, ISSN: 0168-1656, DOI: 10.1016/J.JBIOTEC.2006.05.002 [extrait le 2006-12-01]**

- **WEI XIONG ET AL: "High-density fermentation of microalga Chlorella protothecoides in bioreactor for microbio-diesel production", APPLIED MICROBIOLOGY AND BIOTECHNOLOGY, SPRINGER, BERLIN, DE, vol. 78, no. 1, 6 décembre 2007 (2007-12-06), pages 29-36, XP019586262, ISSN: 1432-0614**
- **LI XIUFENG ET AL: "Large-scale biodiesel production from microalga Chlorella protothecoides through heterotropic Cultivation in bioreactors", BIOTECHNOLOGY AND BIOENGINEERING, WILEY & SONS, HOBOKEN, NJ, US, vol. 98, no. 4, 1 novembre 2007 (2007-11-01), pages 764-771, XP002583837, ISSN: 0006-3592, DOI: 10.1002/BIT.21489 [extrait le 2007-05-11]**

**Description**

La présente invention est relative à un nouveau procédé de production, à l'échelle industrielle, de biomasse de microalgues du genre *Chlorella* riches en lipides de qualité sensorielle acceptable.

La présente invention est particulièrement bien adaptée au contrôle de la rancidité oxydative qui accompagne l'instabilité dans le temps de biomasse de microalgues riches en acides gras monoinsaturés, notamment en acide oléique.

## *Présentation de l'état de l'art*

Ne nécessitant historiquement « que de l'eau et de la lumière du soleil » pour croître, les algues sont considérées depuis longtemps comme une source de nourriture.

Il existe plusieurs espèces d'algues pouvant être utilisées en alimentation, la plupart étant des "macroalgues" comme le varech, la laitue de mer (*Ulva lactuca*) et des algues rouges de type *Porphyra* (cultivée au Japon) ou « dulse » (*Palmaria palmata*).

Cependant, outre ces macroalgues, il y a aussi d'autres sources d'algues représentées par les « microalgues », c'est à dire des algues microscopiques unicellulaires photosynthétiques ou non, d'origine marine ou non, cultivées pour leurs applications dans les biocarburants ou l'alimentation.

Par exemple, la spiruline (*Arthrospira platensis*) est cultivée dans des lagunes ouvertes (par phototrophie) pour une utilisation comme complément alimentaire ou incorporée en petites quantités dans la confiserie ou les boissons (généralement moins de 0,5% poids / poids).

D'autres microalgues riches en lipides, y compris certaines espèces appartenant au genre *Chlorella,* sont aussi très populaires dans les pays asiatiques comme compléments alimentaires.

Plusieurs espèces de microalgues sont capables de passer d'une croissance photo-autotrophe (croissance grâce à la lumière qui fournit l'énergie pour convertir le $CO_2$ en chaînes carbonées) à une croissance hétérotrophe (sans lumière) utilisant le glucose ou d'autres substrats carbonés utilisables pour le métabolisme du carbone et de l'énergie.

Industriellement, trois procédés de production des microalgues sont actuellement utilisés :

- En réacteurs hétérotrophes (entièrement clos) ;
- En bassins à ciel ouvert ;
- En tubes de verre

De ces modes de culture sont produites des Chlorelles aux propriétés et compositions variables. Selon qu'elles sont produites à la lumière ou non, à l'air libre ou pas, leur composition sera différente.

La production et l'utilisation de la farine de microalgues de type *Chlorella* sont par exemple décrites dans les documents WO 2010/120923 et WO 2010/045368, la production par voie hétérotrophe et en l'absence de lumière favorisant leur taux de croissance.

La fraction huile de la farine de microalgues, qui peut être composée essentiellement d'huiles monoinsaturées, peut offrir des avantages nutritionnels et santé par rapport aux huiles saturées, hydrogénées et polyinsaturées souvent trouvées dans les produits alimentaires conventionnels.

Cependant, lorsque l'on souhaite fabriquer industriellement des poudres de farine de microalgues à partir de leur biomasse, d'importantes difficultés demeurent, non seulement du point de vue technologique, mais également du point de vue du profil sensoriel des compositions produites.

En effet, si des poudres d'algues, par exemple fabriquées avec des algues cultivées photosynthétiquement dans des étangs extérieurs ou par photobioréacteurs, sont disponibles dans le commerce, elles ont une couleur vert foncé (liée à la chlorophylle) et un goût fort et désagréable.

Même formulées dans des produits alimentaires ou comme compléments nutritionnels, ces poudres d'algues communiquent toujours cette couleur verte visuellement peu attrayante au produit alimentaire ou au complément nutritionnel et ont un goût désagréable de poisson ou la saveur d'algues marines.

Quant aux chlorelles, le descripteur communément admis dans ce domaine est le goût de « thé vert », un peu semblable à d'autres poudres végétales vertes telles que l'orge vert en poudre ou le blé vert en poudre, goût attribué à sa forte teneur en chlorophylle.

Leur saveur n'est habituellement masquée que lorsqu'elles sont mélangées avec des légumes à saveur forte ou des jus d'agrumes.

Par ailleurs, pour les chlorelles riches en lipides, des saveurs désagréables (terme anglais de « off-notes ») peuvent survenir, liées notamment aux produits de dégradation oxydative des lipides, surtout celle des acides gras monoinsaturés.

C'est ainsi que par exemple l'acide oléique (C18:1) est sensible à l'oxydation, et sa dégradation oxydative conduit à :

- la formation de peroxydes, d'hydroperoxydes et des composés organiques volatils avec une odeur de rance, et
- la perte de la valeur nutritionnelle des acides gras insaturés.

Les produits de dégradation oxydative (enzymatique ou par auto-oxydation) des acides gras insaturés sont principalement des composés carbonyles et des alcools composés de 5 à 9 carbones qui confèrent des odeurs très spécifiques.

Ainsi, les composés à 9 carbones comme le (E,Z)-2,6-nonadiénal, présentent des odeurs de concombres et de melons. Les composés à 8 carbones (I-octen-3-01, 1-octen-3-one, 1,5-octadièn-3-01) contribuent à l'odeur de plantes ou à l'odeur métallique même si individuellement ces composés présentent des odeurs de champignon ou de géranium. Les composés à 6 carbones (hexanol, hexanal, (E)-2-hexénal et (Z)-3-hexen-1-01) participent à l'odeur verte et à l'odeur d'algues. Le (E,E)-2,4-heptadiénal présente quant à lui une odeur verte, de concombre.

Pour contrôler la dégradation oxydative des acides gras à l'échelle du laboratoire, le degré d'aération de la biomasse pour subvenir aux besoins en oxygène de la microalgue est contrôlé par le suivi de la pression en oxygène dissous ($pO_2$).

Le protocole de fermentation comporte alors une régulation de la $pO_2$ réalisée par :

- le débit d'air et/ou
- le débit en oxygène et/ou
- la puissance d'agitation.

Cependant, ce contrôle de la $pO_2$ pose de grandes difficultés lorsqu'il s'agit de transposer le protocole du laboratoire à l'échelle industrielle.

En effet, la $pO_2$ est définie suivant la concentration en oxygène dissous dans le moût de fermentation à saturation. Si de l'eau est mise en aération sous air, à température ambiante et sous pression atmosphérique, suffisamment longtemps, on considère que la $pO_2$ est égale à 100 %.

Or, lors du calibrage d'une sonde $pO_2$ dans un fermenteur, la teneur en oxygène dissous est influencée par la concentration en sels résiduels et par la température de fermentation.

Par ailleurs, il est classiquement admis que pour un fermenteur de laboratoire, la $pO_2$ est peu influencée par la pression générée par la hauteur du moût de fermentation et par les effets de mélange.

Cependant, lors des industrialisations sur des fermenteurs de moyenne (de l'ordre du $m^3$) à grande capacité (de l'ordre de centaines de $m^3$), la hauteur du moût de fermentation va au contraire :

- avoir une influence sur la pression en oxygène dissous ; et
- provoquer des phénomènes complexes dans le fermenteur « non parfaitement agité ».

En ce sens, la valeur de $pO_2$ établie à l'échelle du laboratoire n'est donc pas extrapolable à l'échelle industrielle.

Il existe donc toujours un besoin non satisfait de disposer d'un procédé garantissant la production à l'échelle industrielle de compositions de farine de microalgues du genre *Chlorella* de qualité organoleptique convenable permettant l'utilisation de celles-ci dans des produits alimentaires plus nombreux et diversifiés.

### Résumé de l'invention

La société Demanderesse a trouvé que l'on pouvait remédier à cette difficulté à contrôler la disponibilité en oxygène dissous qui doit être suffisante pour répondre aux besoins de la microalgue tout en évitant au maximum la dégradation oxydative des acides gras monoinsaturés produits, en ajustant lesdits besoins en transfert d'oxygène par le suivi du quotient respiratoire (à l'aide d'un analyseur de gaz), et non pas par le suivi de la réponse d'une sonde de $pO_2$.

Ce contrôle permet par ailleurs :

- de visualiser le comportement métabolique de la souche et
- de palier les problèmes de suroxygénation qui ne peuvent être que constatés classiquement par la mesure de la $pO_2$.

Ainsi, la présente invention concerne un procédé de production fermentaire d'une biomasse, de préférence à l'échelle industrielle, de microalgues riches en lipides comprenant au moins une étape de culture durant laquelle le contrôle de la disponibilité en oxygène dissous dans le fermenteur est assuré par le suivi du quotient respiratoire desdites microalgues.

Les microalgues sont cultivées en conditions hétérotrophiques.

Les microalgues appartiennent au genre *Chlorella* et peuvent être choisies dans le groupe constitué de *Chlorella vulgaris, Chlorella sorokiniana* et *Chlorella protothecoides.* De manière tout particulièrement préférée, les microalgues sont des *Chlorella protothecoides.*

Les microalgues sont riches en lipides. La biomasse obtenue peut notamment présenter une teneur en lipides de plus de 30 ou 40 % en poids sec de biomasse.

L'étape de culture durant laquelle le contrôle de la disponibilité en oxygène dissous dans le fermenteur est assuré par le suivi du quotient respiratoire, est une étape d'accumulation des lipides. En particulier, le contrôle de la disponibilité en oxygène dissous dans le fermenteur peut être assuré par le suivi du quotient respiratoire dès que la biomasse présente une teneur en lipides de plus de 25 %, de préférence plus de 30 % en poids sec de biomasse.

Le procédé selon l'invention permet d'obtenir une biomasse de qualité sensorielle acceptable, notamment comprenant peu ou pas de composés organoleptiquement indésirables tels que les produits de dégradation oxydative des acides gras monoinsaturés, et plus particulièrement les produits de dégradation oxydative de l'acide oléique. L'évaluation de cette qualité sensorielle peut se faire notamment au moyen de descripteurs comprenant la couleur, la texture nappante, la saveur sucrée, et les flaveurs suivantes : champignon, céréales, beurre/produit laitier, huile rance, et arrière-goût végétal.

L'analyse sensorielle peut être réalisée en utilisant une composition de farine de microalgues, comprenant :

- 5-10 % de composition de farine de microalgues, de préférence environ 7%;
- 0,5-2% de sucre, de préférence environ 1% ;
- 0,1-0,5 % d'arôme vanille, de préférence environ 0,25% ; et
- le reste en lait écrémé, de préférence environ 91,75%.

les pourcentages étant en poids de la composition.

ladite composition étant homogénéisée et chauffée à 60-85°C, de préférence environ 75°C, pendant 2-10 minutes, de préférence environ 5 minutes.

L'analyse sensorielle peut également comprendre l'analyse par SPME / GC-MS des composés organiques volatils liées aux descripteurs de l'analyse sensorielle. De préférence, les composés organiques volatils appartiennent aux familles des aldéhydes saturés et di-insaturés, des cétones insaturés, des acides carboxyliques et leurs dérivés.

Afin de prévenir ou réduire la production de ces composés indésirables, le quotient respiratoire est maintenu, durant la phase d'accumulation des lipides lorsque la teneur en lipides est de plus de 25 % en poids sec de biomasse, à une valeur supérieure à 1,5, de préférence supérieure à 1,6, plus préférentiellement supérieure à 1,7 et plus préférentiellement encore supérieure à 1,8.

Le quotient respiratoire peut être suivi au moyen d'un analyseur de gaz et être contrôlé par l'apport d'oxygène dans le milieu de fermentation notamment par la modulation de la vitesse de l'agitation, de la contrepression ou la concentration en oxygène dans l'air entrant (air injecté dans le milieu).

Ce contrôle permet également d'obtenir un ratio métabolique Y $O_2$/S cumulé (calculé depuis le début de la fermentation) maintenu à une valeur inférieure à 0,28, préférentiellement inférieure à 0,27, plus préférentiellement inférieure à 0,26. De manière alternative, il permet également, d'obtenir un ratio métabolique Y $O_2$/S observé durant la phase d'accumulation des lipides, de préférence lorsque la biomasse contient plus de 25 % de lipides, de préférence plus de 30 % de lipides (% exprimé en poids sec de biomasse), maintenu à une valeur inférieure à 0,28, préférentiellement inférieure à 0,27, plus préférentiellement inférieure à 0,26.

### Description détaillée de l'invention

#### Définitions

Au sens de l'invention, une composition de farine de microalgues présente une « qualité sensorielle acceptable », lorsque son évaluation par un panel sensoriel en formulation alimentaire conclut à l'absence de défauts qui altèrent la qualité organoleptique desdites formulations alimentaires contenant ces compositions de farine de microalgues.

Les « défauts » (terme anglo-saxon de « off-notes ») sont associés à la présence de molécules spécifiques odorantes et/ou aromatiques indésirables.

Par « qualité organoleptique » est entendue la propriété d'un aliment en termes de goût, odeur, aspect, couleur et consistance.

Le « quotient respiratoire » correspond au rapport entre la quantité de $CO_2$ produit et la quantité d'$O_2$ consommé par unité de temps. Ce quotient peut être obtenu en analysant les gaz sortant du fermenteur.

Le « ratio métabolique $Y_{O2/S}$ » correspond au rapport entre la quantité d'$O_2$ consommé et la quantité de substrat, en général du glucose, consommé.

« La productivité » correspond à la quantité de biomasse fabriquée par litre et par heure de fermentation en mode fed batch.

« Le rendement de conversion Yx/s » représente classiquement le rapport entre la biomasse formée et le glucose consommé.

« Les microalgues du genre Chlorella » s'entendent ici des microalgues choisies dans le groupe constitué de *Chlorella protothecoides, Chlorella kessleri, Chlorella minutissima, Chlorella sp., Chlorella sorokiniana, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora* et *Prototheca moriformis,* de préférence choisies dans le groupe constitué de *Chlorella vulgaris, Chlorella sorokiniana* et *Chlorella protothecoides. De manière* plus particulièrement préférée, les microalgues sont des *Chlorella protothecoides.*

*Objet de l'invention*

L'homme du métier détermine classiquement la disponibilité en oxygène dissous dans le milieu de fermentation par la mesure de la pression partielle en oxygène ($pO_2$).

Cette technique est satisfaisante sur des petits fermenteurs car la valeur donnée par une sonde de $pO_2$ peut être considérée comme représentative de tout le fermenteur.

Mais elle ne permet pas de connaitre la disponibilité globale en $O_2$ dans un fermenteur industriel de grande taille car la $pO_2$ n'y est pas répartie de façon homogène.

Pour pallier ce problème, la société Demanderesse a donc développé une méthode de contrôle de l'oxygénation par le niveau du quotient respiratoire de la fermentation qui est un indicateur rapide et fiable du métabolisme des microalgues contenues dans le fermenteur.

L'invention est donc relative à un nouveau procédé de production industrielle de biomasse de microalgues du genre *Chlorella,* riche en lipides et sans composés organoleptiquement indésirables. Ce procédé se caractérise par le contrôle de la disponibilité en oxygène dissous à l'aide d'une méthode indirecte (suivi du quotient respiratoire de la souche de microalgue) adaptée à des fermenteurs de grande taille, la notion de méthode indirecte s'opposant à la méthode directe de mesure de la disponibilité en oxygène dissous au moyen d'une sonde mesurant la pression partielle en oxygène ($pO_2$).

Le contrôle de la disponibilité en oxygène dissous par le suivi du quotient respiratoire de la microalgue a pour objectif de réduire la synthèse de molécules responsables de l'apparition des « off-notes » (notamment les produits de dégradations oxydatives des acides gras monoinsaturés tels que l'acide oléique).

En effet, le développement des voies métaboliques conduisant aux molécules indésirables dépend de la disponibilité en $O_2$ dans le milieu de fermentation.

Ces voies de dégradation réclament plus d'oxygène que les voies métaboliques de biosynthèse des molécules d'intérêt, en l'occurrence les voies métaboliques de biosynthèse des acides gras monoinsaturés de type oléique C18:1.

Cela se traduit par un quotient respiratoire plus faible pour ces voies de dégradation que pour la voie de biosynthèse visée.

Ainsi, comme cela est illustré dans les exemples, pour *Chlorella protothecoides* choisie comme microalgue de référence dans le procédé de l'invention, le quotient respiratoire est de 1,8 - ce qui traduit la production de 1,8 moles de $CO_2$ par mole d'$O_2$ consommé - lorsque la voie de biosynthèse de l'acide oléique est majoritaire dans le métabolisme cellulaire.

En revanche, lorsque la fermentation est suroxygénée et que les microalgues génèrent des produits de dégradations oxydatives, le quotient respiratoire ne dépasse pas 1,5.

Le procédé selon l'invention est un procédé de production fermentaire de biomasse riches en lipides, qui comprend une étape de culture durant laquelle le contrôle de la disponibilité en oxygène dissous dans le fermenteur est assuré par le suivi du quotient respiratoire des microalgues.

Les microalgues sont du genre *Chlorella.* En particulier, elles peuvent être choisies dans le groupe constitué de *Chlorella vulgaris, Chlorella sorokiniana* et *Chlorella protothecoides.* De manière préférée, les microalgues sont des *Chlorella protothecoides.*

Bien qu'utilisable à plus petite échelle, le procédé est de préférence mis en œuvre à l'échelle industrielle, c'est-à-dire sur des fermenteurs de moyenne (d'environ 1 à 100 $m^3$) et grande capacité (de plus de 100 $m^3$). Selon un mode de réalisation, le procédé est mis en œuvre sur des fermenteurs d'une capacité d'au moins 1, 10, 25, 50, 75, 100, 500 ou 1000 $m^3$.

Les microalgues sont cultivées en conditions hétérotrophiques, c'est-à-dire sans lumière en utilisant un substrat carboné (de préférence le glucose) comme source de carbone et d'énergie.

La biomasse obtenue par le procédé selon l'invention est une biomasse riche en lipides. L'expression « riche en lipides » telle qu'utilisée dans la présente demande se réfère à une teneur en lipides de plus de 20% en poids sec de biomasse, de préférence de plus de 25%. Selon un mode particulier de réalisation, la biomasse obtenue par le procédé selon l'invention, présente une teneur en lipides de plus de 30, 35, 40 ou 44 % en poids sec de biomasse.

L'étape de culture durant laquelle le contrôle de la disponibilité en oxygène dissous dans le fermenteur est assuré par le suivi du quotient respiratoire des microalgues, est l'étape durant laquelle la biomasse accumule des lipides.

La phase d'accumulation des lipides est la phase de culture durant laquelle la teneur en acides gras de la biomasse augmente. Cette étape peut être consécutive à une étape de croissance destinée exclusivement à augmenter la quantité de biomasse. Elle peut être déclenchée lorsque la quantité de biomasse a atteint un seuil prédéfini, par exemple environ 70 g/L, notamment en remplaçant l'ammoniaque par de la potasse pour réguler le pH.

De manière plus particulièrement préférée, le contrôle de la disponibilité en oxygène dissous dans le fermenteur est assuré par le suivi du quotient respiratoire dès que la teneur en acides gras est supérieure à 25% en poids sec de la biomasse, de préférence supérieure à 30% en poids sec de la biomasse. De manière alternative, le suivi du quotient respiratoire des microalgues peut être effectué durant toute la durée de la fermentation.

Selon un mode préférée, la biomasse obtenue par le procédé selon l'invention est de qualité sensorielle acceptable. Notamment, elle contient peu ou pas de composés organoleptiquement indésirables tels que les produits de dégradation oxydative des acides gras monoinsaturés. En particulier, la biomasse contient peu ou pas de produits de dégradation oxydative de l'acide oléique.

De préférence, la teneur en composés organoleptiquement indésirables demeure inférieure au seuil de détection d'un panel sensoriel.

La teneur en acide linoléique (produit de dégradation oxydative de l'acide oléique) est inférieure à 18, 17, 16, 15, 14, 13, 12, 11, 10, 9, 8 ou 7% en poids par rapport au poids total des acides gras dans la biomasse sèche.

La qualité sensorielle de la biomasse peut être définie en préparant une composition de dégustation de farine de microalgues et en demandant à un panel, de préférence d'au moins 10 personnes, d'évaluer des descripteurs relatifs à l'aspect, la texture, les saveurs et les flaveurs.

La farine de microalgues peut être préparée à partir de biomasse broyées et séchées par toute méthode connue de l'homme du métier, telle que par exemple celle décrite dans la demande de brevet WO 2010/12093. Par «composition de farine de microalgues » est entendu une composition comprenant au minimum 50, 60, 70, 80, 90 ou 95 % en poids sec de biomasse de microalgues. Cependant, d'autres ingrédients peuvent facultativement être compris dans cette composition. La biomasse de microalgues est dérivée de cellules de microalgues, qui peuvent être entières ou cassées, ou un mélange de cellules entières et cassées.

La société Demanderesse a par ailleurs défini dans la demande de brevet français n°13 56113, une matrice de dégustation très simple qui permet de faire une évaluation organoleptique similaire à celle obtenue avec des recettes beaucoup plus complexes et très différentes telles qu'une crème glacée ou une brioche. L'évaluation avec cette matrice de dégustation est beaucoup plus précise ou exacte que celle faite avec une simple solution aqueuse, qui s'est révélée être incapable de prédire les qualités organoleptiques des compositions de farine de microalgues dans une glace, par exemple.

Ainsi, de préférence, la composition de dégustation de farine de microalgues utilisée pour évaluer les qualités sensorielles de la biomasse comprend :

- 5-10 % de composition de farine de microalgues, de préférence environ 7 % ;
- 0,5-2% de sucre, de préférence environ 1% ;
- 0,1-0,5 % d'arôme vanille, de préférence environ 0,25% ; et
- le reste en lait écrémé, de préférence environ 91,75%.

les pourcentages étant exprimés en poids de la composition.

Cette composition est obtenue par homogénéisation puis chauffage à 60-85°C, de préférence environ 75°C, pendant 2-10 minutes, de préférence environ 5 minutes.

Les descripteurs de la composition sont évalués par comparaison avec une composition de référence, c'est-à-dire une composition de dégustation obtenue à partir d'une farine de microalgues de référence identifiée comme conforme, c'est-à-dire de qualité organoleptique acceptable (perçue conforme par ledit panel dans l'ensemble des descripteurs testés).

De préférence, les produits de référence tels que présentés dans le tableau suivant sont associés à chaque descripteur :

| Descripteurs | | Référence |
|---|---|---|
| Aspect | Couleur (de clair à foncé) | |
| Texture | Nappant | lait entier + 5% crème |
| Saveurs | Sucré | Saccharose 1% |

(suite)

| Descripteurs | | Référence |
|---|---|---|
| Flaveurs | Champignon | 100 g de champignons dans 100 ml d'eau froide / dilution X 4 |
| | Céréales | Solution Ebly 10% |
| | Beurre / produit laitier | |
| | Huile rance | Huile oxydée 1,5% |
| | Arrière-goût végétal | Composition de farine de microalgues très inacceptable |

A chaque séance de dégustation les produits sont évalués sur chaque descripteur en comparaison du lot de référence considéré comme de qualité organoleptique acceptable.

Tous les produits sont évalués les uns après les autres, sur des échelles allant par exemple de 1 à 9 de la manière suivante :

Valeur de 1 :    le descripteur évalué n'est pas présent dans le produit ;
Valeur de 5 :    le descripteur évalué est présent dans le produit exactement de la même façon que sur le produit de référence de qualité organoleptique acceptable ;
Valeur de 9 :    le descripteur évalué est très présent dans le produit.

Il est important de noter que le lot de référence de qualité organoleptique acceptable n'est pas forcément la farine de microalgues présentant le profil sensoriel optimal : il s'agit de préférence d'une composition de farine de microalgues perçue par le panel sensoriel comme "satisfaisant", notamment présentant une note de 5 sur tous les descripteurs testés.

La société Demanderesse a par ailleurs établi que le profil sensoriel d'une composition de farine de microalgues pouvait être également défini par la nature et le seuil de détection de molécules spécifiques odorantes, notamment de composés organiques volatils particuliers (cf. la demande de brevet français n°13 56113).

En effet, elle a identifié un ensemble de 13 composés organiques volatils dont la teneur globale dans une composition de farine de microalgues permet de déterminer la qualité organoleptique de celle-ci.

Ces 13 composés organiques volatils sont les suivants : l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone.

De préférence, ces composés organiques volatils sont prélevés par micro extraction en phase solide (SPME) et analysés par chromatographie en phase gazeuse GC, en particulier par GC-MS (chromatographie en phase gazeuse couplée à la spectrométrie de masse).

La teneur en chacun des 13 composés organiques volatils est déterminée par la surface du pic de l'ion spécifique du chromatogramme SPME-GC/MS correspondant à ce composé organique volatile et est déterminée en comparaison à celle d'un produit de référence.

La teneur totale est obtenue en additionnant les teneurs de chacun des 13 composés, de préférence en déterminant la surface totale des pics de chromatographie correspondant aux 13 composés. La teneur totale peut ensuite être comparée à celle d'une composition de farine de microalgues de référence pour laquelle les qualités organoleptiques sont définies comme acceptables ou inacceptables.

Ainsi, une faible teneur totale en ces 13 composés organiques volatils est associée à une qualité organoleptique optimisée. A contrario, une teneur totale plus élevée en ces 13 composés organiques volatils est associée à une qualité organoleptique moyenne, voire mauvaise ou inacceptable.

Selon un mode particulier de réalisation, la composition de farine de microalgues obtenues avec le procédé selon l'invention, a une teneur totale en ces 13 composés organiques volatils qui est au minimum deux fois inférieure à celle d'une composition d'une qualité organoleptique inacceptable, de préférence au minimum 5, 10 ou 15 fois inférieure.

Selon un autre mode particulier de réalisation, la composition de farine de microalgues obtenues avec le procédé selon l'invention, a une teneur totale en ces 13 composés organiques volatils qui est identique ou inférieure à celle d'une composition d'une qualité organoleptique acceptable.

Lors de l'étape de culture durant laquelle le quotient respiratoire est suivi, celui-ci est maintenu à une valeur supérieure à 1,5, de préférence supérieure à 1,6, plus préférentiellement supérieure à 1,7 et plus préférentiellement encore supérieure à 1,8.

Ainsi, dans un mode particulier de réalisation, les conditions de fermentation de la microalgue, de préférence *Chlorella protothecoides,* sont contrôlées de manière à maintenir le quotient respiratoire à une valeur supérieure à 1,5, de préférence supérieure à 1,6, plus préférentiellement supérieure à 1,7 et plus préférentiellement encore supérieure à 1,8, lors de l'étape d'accumulation des lipides, et plus particulièrement dès que la biomasse produite contient plus de 25 % de lipides, de préférence plus de 30 % de lipides (% exprimé en poids sec de biomasse).

Le quotient respiratoire peut être suivi de manière continue ou semi-continue au moyen d'un analyseur de gaz qui analyse les gaz s'échappant du fermenteur, notamment qui quantifie le $CO_2$ et l'$O_2$.

Sur la base de ces mesures, il est alors possible d'ajuster l'apport en oxygène afin de moduler le quotient respiratoire des microalgues et ainsi contrôler efficacement leur métabolisme.

Ainsi, dans le cas où le quotient respiratoire est trop faible, l'apport en oxygène est diminué. Dans le cas contraire, l'apport en oxygène peut être augmenté ou demeuré inchangé.

L'apport en oxygène dans le milieu de fermentation peut se faire par tout moyen, notamment par la modulation de la vitesse de l'agitation, de la contrepression ou de la concentration en oxygène dans l'air entrant (air injecté dans le milieu).

La société Demanderesse a en outre observé que le ratio métabolique $Y_{O2/S}$ (exprimant la quantité d'oxygène consommé / quantité de glucose consommé) peut être utilisé pour déterminer si l'oxygénation se situe dans la bonne gamme.

Ainsi, selon un mode préféré, le ratio métabolique $Y O_2/S$ cumulé (calculé depuis le début de la fermentation) est maintenu à une valeur inférieure à 0,28, préférentiellement inférieure à 0,27, plus préférentiellement inférieure à 0,26, en particulier lorsque la microalgue est une *Chlorella protothecoides* et que la biomasse produite par le procédé selon l'invention présente une teneur en lipides de plus de 30% en poids sec de biomasse.

Selon un autre mode préféré, le ratio métabolique $Y O_2/S$ observé durant la phase d'accumulation des lipides, notamment lorsque la biomasse produite contient plus de 25 % de lipides, de préférence plus de 30 % de lipides (% exprimé en poids sec de biomasse), est maintenu à une valeur inférieure à 0,32, préférentiellement inférieure 0,28, plus préférentiellement inférieure à 0,27, et de manière tout particulièrement préférée inférieure à 0,26.

Selon un autre aspect, la présente demande décrit également un procédé de culture hétérotrophique de microalgues comprenant :

- une première étape de culture permettant la croissance des microalgues, et
- une deuxième étape de culture permettant d'enrichir la biomasse en lipides et durant laquelle le contrôle de la disponibilité en oxygène dissous dans le fermenteur est assuré par le suivi du quotient respiratoire desdites microalgues.

De préférence, dans la deuxième étape, le contrôle de la disponibilité en oxygène dissous est assuré par le suivi du quotient respiratoire dès que la teneur en acides gras est supérieure à 25% en poids sec de la biomasse, de préférence supérieure à 30% en poids sec de la biomasse.

Les modes de réalisation décrits ci-dessus s'appliquent également à cet aspect.

Selon un autre aspect, la présente demande décrit également un procédé de production d'une composition de farine de microalgues caractérisé en ce qu'il comprend la production de la biomasse de microalgues selon le procédé de l'invention et l'obtention d'une farine de microalgues à partir de ladite biomasse.

L'invention sera mieux comprise à l'aide des exemples qui suivent, lesquels se veulent illustratifs et non limitatifs.

**EXEMPLES**

**Exemple 1** : **Production de *Chlorella protothecoides* riches en lipides** - **Oxygénation contrôlée**

La souche utilisée est *Chlorella protothecoides UTEX250*

Préculture :

- 500 mL de milieu dans un Erlenmeyer de 2L ;
- Composition du milieu:

Tableau 1.

| | | |
|---|---|---|
| Macro éléments (g/L) | Glucose | 40 |
| | $K_2HPO_4$ | 3 |
| | $Na_2HPO_4$ | 3 |
| | $MgSO_4.7H_2O$ | 0,25 |
| | $(NH_4)_2SO_4$ | 1 |
| | Acide Citrique | 1 |
| | clerol FBA 3107 (antimousse) | 0,1 |
| Micro-éléments et Vitamines (mg/L) | $CaCl_2.2H_2O$ | 30 |
| | $FeSO_4.7H_2O$ | 1 |
| | $MnSO_4.1H_2O$ | 8 |
| | $CoSO_4.7H_2O$ | 0,1 |
| | $CuSO_4.5H_2O$ | 0,2 |
| | $ZnSO_4.7H_2O$ | 0,5 |
| | $H_3BO_3$ | 0,1 |
| | $Na_2MoO_4 \cdot 2H_2O$ | 0,4 |
| | Thiamine HCl | 1 |
| | Biotine | 0,015 |
| | B12 | 0,01 |
| | pantothénate de calcium | 0,03 |
| | acide p-aminobenzoique | 0,06 |

L'incubation se déroule dans les conditions suivantes : durée : 72 h ; température : 28°C ; agitation : 110 rpm (Incubateur Infors Multitron).
La préculture est ensuite transférée dans un fermenteur de 30L de type Sartorius.

Culture pour production de biomasse :

Le milieu de base est le suivant :

Tableau 2.

| | | | |
|---|---|---|---|
| **Macro éléments (g/L)** | Glucose | | 40 |
| | KH$_2$PO$_4$ | | 0,9 |
| | NaH$_2$PO$_4$ | | 0,7 |
| | MgSO$_4$.7H$_2$O | | 1,7 |
| | (NH$_4$)$_2$SO$_4$ | | 0,2 |
| | clerol FBA 3107 (antimousse) | | 0,3 |
| **Micro-éléments et Vitamines (mg/L)** | CaCl$_2$.2H$_2$O | | 20 |
| | FeSO$_4$.7H$_2$O | | 6 |
| | MnSO$_4$.1H$_2$O | | 20 |
| | CoSO$_4$.7H$_2$O | | 0,05 |
| | CuSO$_4$.5H$_2$O | | 0,3 |
| | ZnSO$_4$.7H$_2$O | | 25 |
| | H$_3$BO$_3$ | | 7 |
| | Na$_2$MoO$_4$ · 2H$_2$O | | 1 |
| | Inositol | | 100 |
| | Chlorure de Choline | | 100 |
| | Thiamine HCl | | 3 |
| | Biotine | | 0,05 |
| | B12 | | 0,03 |
| | pantothénate de calcium | | 0,1 |
| | acide p-aminobenzoique | | 0,1 |

Le volume initial (Vi) du fermenteur est ajusté à 7 L après ensemencement. Il est porté à 15 - 20 L en final.

Les paramètres de conduite des différents essais sont les suivants :

Tableau 3.

| | |
|---|---|
| Température | 28 °C |
| pH | 5.2 par NH$_3$ 28% w/w puis KOH 5N |
| QR | **Essai 1 : protocole de Base**<br>pO$_2$ = 30% ± 5% (maintenue par agitation)<br>**QR = 1,7 - 1,9**<br>Gaz entrant = air |
| | **Essai 2 : Suroxygénation**<br>pO$_2$ = 120% ± 20% (maintenue par agitation)<br>**QR = 1,4 - 1.5**<br>Gaz entrant = 90% air + 10 % d'oxygène pur |
| | **Essai 3 : Sous oxygénation**<br>**pO$_2$ = 0%**<br>**QR = 1,8 - 1,9**<br>**Gaz entrant = air**<br>**Agitation maintenue 10 % en dessous du niveau de l'essai 1.** |
| Agitation minimale | 300 RPM mini |
| Débit de gaz | 15 L/min |

Lorsque la concentration résiduelle en glucose tombe en dessous de 10 g/L, un apport de glucose sous forme d'une solution concentrée à 700 g/L est réalisé en continu de façon à maintenir la teneur en glucose entre 0 et 20 g/L dans le fermenteur.

Lorsque 1000 g de glucose ont été consommés et que la biomasse a atteint une concentration de 70 g/L, l'ammoniaque est remplacé par de la potasse pour la régulation de pH. Cela permet à la biomasse d'accumuler des lipides.

Résultats:

Les essais ont été réalisés avec trois niveaux d'oxygénation. La teneur en oxygène dissous mesurée dans le fermenteur ($pO_2$) qui est exprimée en pourcentage de la teneur obtenue à saturation lorsque le fermenteur est maintenu à pression atmosphérique et alimenté par de l'air. Une $pO_2$ de 100 % correspond à une teneur en oxygène de 7 mg / L environ.

Deux indicateurs métaboliques sont utilisés pour quantifier l'impact sur le métabolisme du niveau d'oxygénation :

- Le Quotient Respiratoire (QR) = $CO_2$ produit / $O_2$ consommé

- Le rendement $Y_{O2/S}$ = $O_2$ consommé / glucose consommé.

Ils sont calculés à partir des teneurs en oxygène et en $CO_2$ du gaz sortant du fermenteur, mesurées à l'aide d'un analyseur de gaz.

Les acides gras ont été quant à eux déterminés par chromatographie en phase gazeuse sous la forme d'esters méthyliques après transestérification au méthanol chlorhydrique et extraction au chloroforme. Les résultats sont exprimés en distribution, en % ; l'analyse est réalisée par la méthode de normalisation interne.

Un chromatographe (Type VARIAN 3800) équipé d'un injecteur « split-splitless » muni d'un « tapfocus liner » et d'un détecteur à ionisation de flamme a été utilisé.

Une solution d'étalon interne à environ précisément 0.5 mg de méthylheptadécanoate par ml de méthanol a été préparée. Le méthylheptadécanoate a servi de repère chromatographique.

Dans un tube de 6 ml, on a pesé environ précisément 30 mg d'échantillon séché au préalable. On a ajouté à la pipette à 2 traits 1 ml de la solution d'étalon interne puis 2 ml de méthanol chlorhydrique 3N. On a ensuite bouché et placé au bain à sec thermostaté à 110°C pendant 4 h.

Après refroidissement, on a ajouté environ 0.5 ml d'eau et 0.5 ml d'eau saturée en chlorure de sodium, extraire par 3 fois 1 ml de chloroforme. On a récupéré les phases chloroformiques dans un tube de 6 ml en les séchant sur une colonne contenant du sulfate de sodium. On a concentré sous courant d'azote jusqu'à 1 mL environ et injecté.

La distribution de chaque acide gras (i), en %, a été obtenu par le ratio de la surface du pic de cet acide gras par rapport à la somme des surfaces de tous les pics repérés sur le chromatogramme, de l'acide laurique (C12:0) au DHA (C22:6 Δ4c, 7c, 10c, 13c, 16c, 19c) inclus, en excluant le pic du méthylheptadécanoate.

Tableau 4.

| Essai | Oxygénation | $pO_2$ (%) | Durée (h) | Biomasse (g/L) | % Acides gras | QR final | $Y_{O2/S}$ Final (g/g) | $Y_{O2/S}$ Cumulé (g/g) | % acide linoléique / Σ AG |
|---|---|---|---|---|---|---|---|---|---|
| 1 | Base | 30 | 93,5 | 182 | 44,6 | 1,82 | 0,27 | 0,26 | 13,6 |
| 2 | Excès | 120 | 95,3 | 186 | 39,3 | 1,47 | 0,33 | 0,29 | 19,6 |
| 3 | Limitante | 0 | 99,1 | 188 | 43,2 | 1,86 | 0,22 | 0,24 | 6,3 |

L'augmentation de l'oxygénation entraine une diminution du quotient respiratoire (augmentation de la consommation d'oxygène par rapport à la production de $CO_2$) et une augmentation du ratio métabolique $Y_{O2/S}$ (augmentation de la consommation d'oxygène par rapport à la consommation de glucose).

Cela s'explique par le développement des voies de désaturation des acides gras qui réclament plus d'oxygène que la synthèse des acides gras saturés.

En conditions d'oxygénation limitée, ces voies de désaturation sont peu développées comme le montre la faible proportion d'acide linoléique dans la biomasse obtenue et la valeur du quotient respiratoire (1,86) observée en fin de fermentation est proche de la valeur théorique de la voie de synthèse de l'acide oléique :

$$35/6\ C_6H_{12}O_6 + 9{,}5O_2 \rightarrow C_{18}H_{34}O_2 + 17CO_2 + 18H_2O$$

Inversement, en condition de suroxygénation, comme dans l'essai 2 où le quotient respiratoire est maintenu à une valeur inférieure à 1,5, soit nettement inférieur à la valeur optimale, on observe une accumulation d'acide linoléique dans la biomasse (19,6 %) ainsi que l'apparition d'off-notes (saveur rance prononcée détectée par un panel sensoriel).

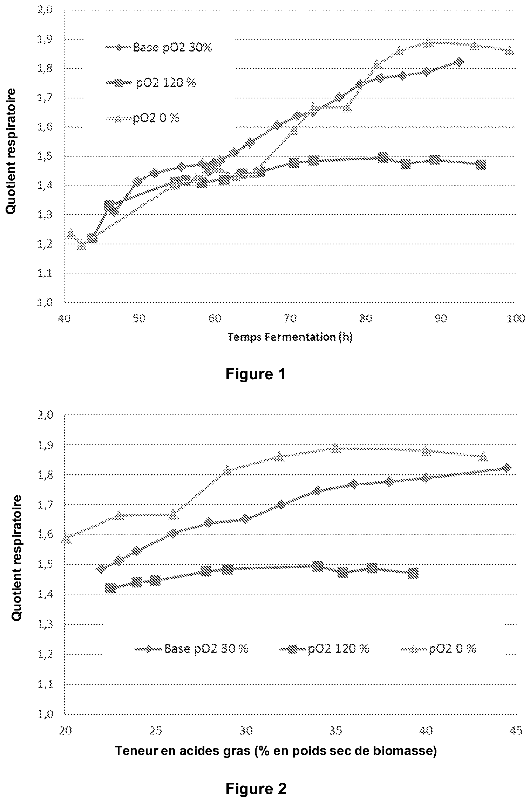

Les graphes des figures 1 et 2 montrent l'évolution des quotients respiratoires des trois essais en fonction du temps et de la teneur en acide gras (en poids sec de biomasse). Ces deux représentations montrent que la suroxygénation se traduit par un blocage de la valeur du quotient respiratoire en deçà de 1,5. En revanche, lorsque les conditions d'oxygénation sont correctes, le quotient respiratoire durant l'étape d'accumulation des lipides, notamment lorsque la teneur en acides gras est d'au minimum 25% en poids sec de biomasse, est supérieur à 1,5.

**Exemple 2 : Evaluation sensorielle des 3 lots produits dans l'exemple 1**

Dans cet exemple, la société Demanderesse se propose de déterminer la qualité sensorielle de farines de microalgues préparées à partir des 3 lots produits dans l'exemple 1, farine préparées à partir de biomasses broyées et séchées selon la méthode décrite dans la demande de brevet WO 2010/12093.

La société Demanderesse a défini dans la demande de brevet français n°13 56113, une matrice de dégustation très simple qui permet de faire une évaluation organoleptique similaire à celle obtenue avec des recettes beaucoup plus complexes et très différentes telles qu'une crème glacée ou une brioche. L'évaluation avec cette matrice de dégustation est beaucoup plus précise ou exacte que celle faite avec une simple solution aqueuse, qui s'est révélée être incapable de prédire les qualités organoleptiques des compositions de farine de microalgues dans une glace, par exemple.

La composition de dégustation de farine de microalgues comprend ainsi :

- 7 % de composition de farine de microalgues ;
- 1% de sucre ;
- 0,25 % d'arôme vanille ; et
- 91,75% de lait écrémé,

les pourcentages étant exprimés en poids de la composition.

Cette composition est ensuite homogénéisée puis chauffée à 75°C pendant 5 minutes.

Un ensemble de 15 personnes est rassemblé pour évaluer des descripteurs de plusieurs compositions de farine de microalgues en comparaison d'un échantillon de farine de microalgues de référence identifié conforme, c'est-à-dire de qualité organoleptique acceptable (lot de référence), i.e. un lot de farine de microalgues perçu conforme par ledit panel dans l'ensemble des descripteurs testés, standard interne au panel.

Les produits de référence tels que présentés dans le tableau 5 suivant sont associés à chaque descripteur :

Tableau 5.

| Descripteurs | | Référence |
|---|---|---|
| Aspect | Couleur (de clair à foncé) | |
| Texture | Nappant | lait entier + 5% crème |
| Saveurs | Sucré | Saccharose 1% |
| Flaveurs | Champignon | 100 g de champignons dans 100 ml d'eau froide / dilution X 4 |
| | Céréales | Solution Ebly 10% |
| | Beurre / produit laitier | |
| | Huile rance | Huile oxydée 1,5% |
| | Arrière-goût végétal | Composition de farine de microalgues très inacceptable |

A chaque séance de dégustation les produits sont évalués sur chaque descripteur en comparaison du lot de référence considéré comme de qualité organoleptique acceptable.

Tous les produits sont évalués les uns après les autres, sur des échelles allant de 1 à 9 de la manière suivante :

Valeur de 1 : le descripteur évalué n'est pas présent dans le produit ;

Valeur de 5 : le descripteur évalué est présent dans le produit exactement de la même façon que sur le produit de référence de qualité organoleptique acceptable ;

Valeur de 9 : le descripteur évalué est très présent dans le produit.

Le lot de référence de qualité organoleptique acceptable est une composition de farine de microalgues perçue par le panel sensoriel comme "satisfaisant", c'est-à-dire présentant une note de 5 sur tous les descripteurs testés.

*Logiciel de traitement des données*

Les analyses ont été réalisées à l'aide du logiciel R (en vente libre) :

R version 2.14.1 (2011-12-22)
Copyright (C) 2011 The R Foundation for Statistical Computing
ISBN 3-900051-07-0
Platform: i386-pc-mingw32/i386 (32-bit)

Le logiciel est un environnement de travail qui nécessite le chargement de modules contenant les fonctions de calcul.

Les modules utilisés dans cette étude sont les suivants :

- Pour l'ACP : Package *FactoMineR* version 1.19
- Pour l'ANOVA : Package car version 2.0-12
- Pour la Régression Linéaire : Package *stats* version 2.14.1

*Traitement des données :*

Des analyses de variances (ANOVA) sont réalisées pour évaluer le pouvoir discriminant des descripteurs (descripteurs dont la p-value associée au test de Fisher - ANOVA de type 1 - est inférieure à 0,20 pour l'effet Composition dans le modèle *descripteur - composition + juge*).

L'effet « composition » s'interprète comme le pouvoir discriminant des descripteurs : s'il n'y a pas d'effet (Probabilité Critique > 0,20), les compositions n'ont pas été discriminées selon ce critère. Plus la probabilité critique est petite, plus le descripteur est discriminant.

Une Analyse en Composantes Principales (ACP) est ensuite réalisée afin d'obtenir une cartographie sensorielle des compositions, ainsi qu'une représentation simultanée de toutes les compositions sur tous les descripteurs.

Les 3 lots de l'exemple 1 ont été analysés selon la méthode décrite ci-dessus.

On présente ici deux exemples sur les descripteurs "beurre / produits laitiers" et "arrière-goût végétal".

"Arrière-goût végétal"

Tableau d'analyse de variance

| | Df | Somme Sq | Moyenne Sq | F value | **Pr(>F)** |
|---|---|---|---|---|---|
| **Composition** | 9 | 109,693 | 12.1881 | 18,2423 | **< 2e-16** |
| Juge | 13 | 18,732 | 1,4409 | 2,1566 | 0,01298 |
| Résidus | 185 | 123,603 | 0,6681 | --- | |

"Beurre / produits laitiers"

Tableau d'analyse de variance

| | Df | Somme Sq | Moyenne Sq | F value | **Pr(>F)** |
|---|---|---|---|---|---|
| **Composition** | 9 | 8,292 | 0,92131 | 1,4530 | **0.1699** |
| Juge | 13 | 8,235 | 0,63347 | 0,9991 | 0,4547 |
| Résidus | 160 | 101,451 | 0,63407 | --- | |

Il apparait que les probabilités critiques associées à l'effet composition pour les 2 descripteurs étudiés sont inférieures à 0,2 : les 2 descripteurs sont donc discriminants. La probabilité critique est plus petite sur le descripteur « arrière-goût végétal » que sur le descripteur « beurre / produits laitiers » ce qui signifie qu'on observe plus de différence entre les compositions sur le premier critère que sur le deuxième.

Voici un tableau récapitulant les probabilités critiques obtenues pour les effets composition et juge pour tous les descripteurs.

Tableau 6.

|  | Composition | juge |
|---|---|---|
| Couleur jaune | 0,00 | 0,05 |
| arrière-goût végétal | 0,00 | 0,31 |
| Goût huile rance | 1 | 1 |
| Nappant | 0,60 | 0,03 |
| Céréales | 0,03 | 0,36 |
| Champignons | 0,12 | 0,01 |
| Sucré | 0,50 | 0,09 |
| Produits laitiers | 0,59 | 0,87 |

Les descripteurs jaune, arrière-goût végétal, céréales et champignon sont discriminants, on les garde tous pour établir l'ACP.

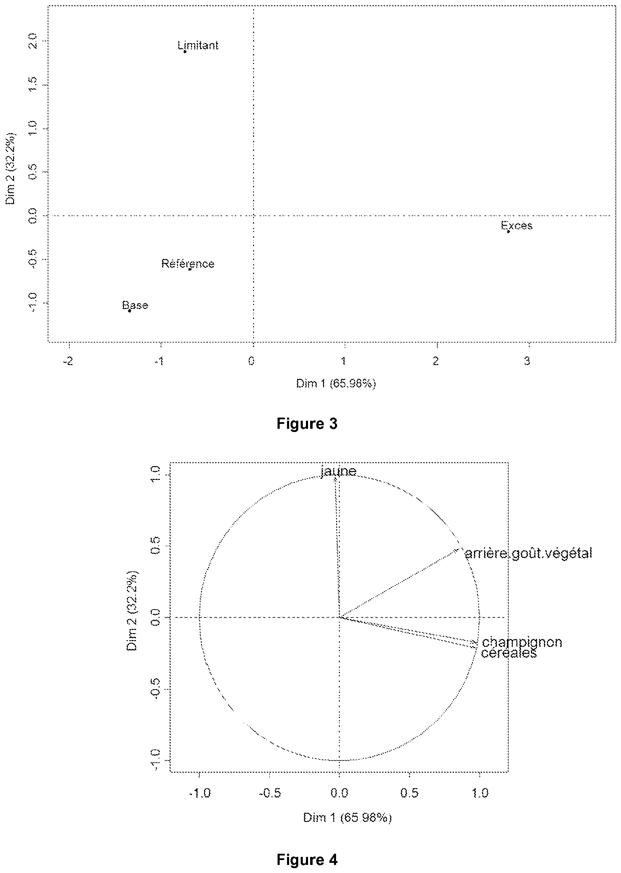

Puisque l'aromatique est un critère essentiel des compositions, l'ACP a été réalisée sur les descripteurs relatifs aux arômes uniquement (champignon, céréales, arrière-goût végétal, produit laitier, rance). La représentation graphique de cette ACP est fournie dans les Figure 3 et Figure 4.

Cette méthode permet d'établir un classement de la qualité organoleptique des différentes farines de microalgues produites à partir des 3 essais de l'exemple 1. Ainsi, les farines obtenues à partir des essais 1 et 3 (oxygénation de base ou limitante) ont des qualités organoleptiques similaires qui sont bien supérieures à celles des farines obtenues à partir de l'essai 2 (oxygénation en excès).

En effet, les conditions d'oxygénation définies comme « en excès » entrainent des flaveurs champignon et céréales plus importantes ainsi qu'un arrière-goût végétal. Ces conditions sont donc à proscrire pour garantir la neutralité du goût du produit.

Les conditions d'oxygénation définies comme « limitantes » fournissent un produit un peu plus jaune mais peu différent en termes de goût du produit obtenu dans les conditions d'oxygénation définies comme « de base », lui-même très proche du produit de référence.

**Exemple 3 : Analyse des composés organiques volatils (VOC) liés aux classifications organoleptiques inacceptables « off-notes » dans les 3 lots de compositions de farine de microalgues obtenus à partir de l'exemple 1**

Pour réaliser l'analyse en SPME / GC-MS des 3 lots de compositions de farine de microalgues obtenus à partir des biomasses produites dans l'exemple 1, on procède comme suit.

Une prise d'essai de 3 g d'échantillon est introduite dans un flacon SPME (20 ml) scellé et incubée à 60°C pendant 15 min puis extraite à 60°C pendant 45 min avec une fibre SPME en DVB/CAR/PDMS (abréviation anglo-saxonne pour divinylbenzene / carboxen / polydimethylsiloxane, df 50/30 $\mu$m).

Les composés organiques volatils extraits sont désorbés dans l'injecteur du système GC-MS TSQ de Thermo Scientific, puis séparés sur une colonne CPwax52 (60m * 0.25 mm, 0.25 $\mu$m) avec le gaz Hélium à 1.5 ml/min.

Le programme de température est : 50°C isotherme pendant 3 min, puis programmation à 5°C/minute jusque 230°C puis isotherme pendant 20 min.

La détection est faite par spectrométrie de masse en mode impact électronique (EI) et les composés sont identifiés par comparaison avec les spectres EI de la bibliothèque NIST.

Comme indiqué plus haut, la société Demanderesse a identifié dans la demande de brevet français n°13 56113, 13 composés permettant de définir la qualité organoleptiques des farines de microalgues obtenues selon l'invention.

Ces 13 composés sont les suivants: heptanal, 3-octène-2-one, 2,4-heptadiénal, 3,5-octadiène-2-one, 2,4-nonadiénal, 2,4-décadiénal, acide hexanoïque, acide 2-éthylhexanoïque, acide heptanoïque, myristate-1, laurate-1, myristate-2, et géranyl-acétone. On retrouve donc ici représentées les familles des aldéhydes di-insaturés, des cétones insaturés, des acides carboxyliques et des dérivés d'acides carboxyliques.

Les seuils olfactifs dans l'eau, attribués à ces 13 composés sont présentés dans le tableau 7 suivant.

Tableau 7.

|  | seuil olfactif dans l'eau (ppb) |
|---|---|
| 2,4-décadiénal | 0,07 |
| 2,4-heptadiénal* | 0,1 |
| 2,4-nonadiénal | 0,01 |
| 3,5-octadiène-2-one* | 1 |
| 3-octène-2-one* | 1 |
| Heptanal | 3 |
| Acide 2-éthylhexanoïque * | 1000 |
| Acide heptanoïque | 3000 |
| Acide hexanoïque | 3000 |
| géranyl-acétone | 60 |
| myristate-1 * | 1 |
| laurate-1 * | 1 |
| myristate-2* | 1 |
| (*) seuil olfactif établi par la société Demanderesse | |

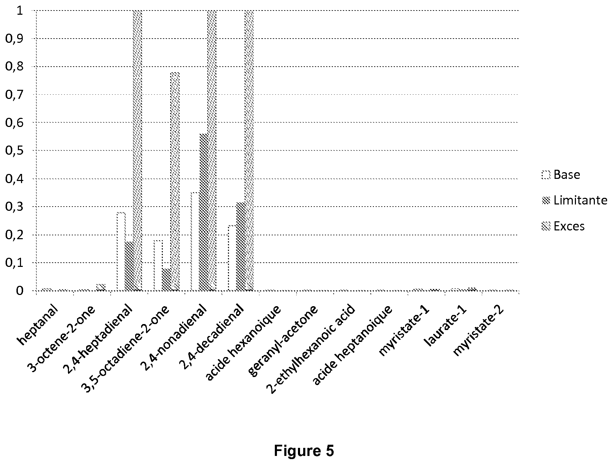

La Figure 5 présente les valeurs d'arômes individuelles de chacun des 13 composés pour les 3 lots de l'exemple 1.

Il apparaît que :

- 4 composés principaux totalisent l'ensemble des arômes détectés : le 2,4 heptadiénal, le 3,5 octadiène-2-one, le 2,4-nonadienal et le 2,4-décadienal, ce qui est conforme à ce qui est connu dans l'état de l'art comme produit de dégradation oxydative (peroxydation) des acides gras insaturés ;
- la teneur en 2,4 heptadiénal et en 3,5 octadiène-2-one est relativement plus faible pour les lots « base » et « limitant » par rapport au lot « excès », le lot « limitant » présentant la teneur la plus faible ;
- la teneur en 2,4-nonadienal et en 2,4-décadienal est relativement plus faible pour les lots « base » et « limitant » par rapport au lot « excès », le lot « base » présentant la teneur la plus faible.

Ces résultats traduisent une meilleure qualité organoleptique des lots « limitant » et « base ».

Le tableau 8 suivant présente la somme des valeurs d'arômes individuelles de ces 13 composés, donc la valeur d'arôme totale, déterminée à partir des teneurs relatives des 13 composés et leurs seuils olfactifs:

$$VA\ total = \Sigma VAx\ \text{(somme des VA individuelles)},$$

avec *VAx = Concentration du composé x / seuil olfactif du composé x)* pour chacun des lots de compositions de farine de microalgues (valeur de 100 % attribuée au lot « Excès »).

Tableau 8.

|  | Valeur d'arôme totale (%) |
|---|---|
| Base | 5,6 |
| Excès | 100 |
| Limitant | 6 |

Ici encore, cette analyse par GC/MS permet également de démontrer que les farines obtenues à partir des essais 1 et 3 (oxygénation de base ou limitante) de l'exemple 1 ont des qualités organoleptiques similaires qui sont bien supérieures à celles des farines obtenues à partir de l'essai 2 (oxygénation en excès).

**Description des Figures**

**FIGURE 1** : Evolution des quotients respiratoires en fonction du temps de fermentation (en heures) pour les essais 1 (Base pO$_2$ 30%), 2 (pO$_2$ 0%) et 3 (pO$_2$ 120%).
**FIGURE 2** : Evolution des quotients respiratoires en fonction de la teneur en acides gras pour les essais 1 (Base pO$_2$ 30%), 2 (pO$_2$ 0%) et 3 (pO$_2$ 120%).
**FIGURE 3 :** Représentation graphique des différents lots (nuage de points) de l'ACP.
**FIGURE 4** : Cercle de corrélation de l'ACP représentant les profils aromatiques des différents lots.
**FIGURE 5** : Valeurs d'arômes individuelles des 3 lots de production.

**Revendications**

1. Procédé de production fermentaire de biomasse de microalgues riches en lipides du genre *Chlorella,* **caractérisé en ce que** les microalgues sont cultivées en conditions hétérotrophiques et **en ce qu'**il comprend au moins une étape de culture durant laquelle la biomasse accumule des lipides et présente une teneur en lipides de plus de 25 % en poids sec de biomasse, durant laquelle le contrôle de la disponibilité en oxygène dissous dans le fermenteur est assuré par le suivi du quotient respiratoire desdites microalgues, et durant laquelle le quotient respiratoire est maintenu à une valeur supérieure à 1,5, la teneur en acide linoléique dans la biomasse produite étant inférieure à 18% en poids par rapport au poids total des acides gras dans la biomasse sèche.

2. Procédé selon l'une quelconque des revendications précédentes, **caractérisé en ce que** les microalgues sont choisies dans le groupe constitué de *Chlorella vulgaris, Chlorella sorokiniana* et *Chlorella protothecoides.*

3. Procédé selon l'une quelconque des revendications précédentes, **caractérisé en ce que** les microalgues sont *Chlorella protothecoides.*

4. Procédé selon l'une quelconque des revendications précédentes, **caractérisé en ce que** la biomasse produite par ledit procédé présente une teneur en lipides de plus de 30% en poids sec de biomasse, de préférence de plus de 40%.

5. Procédé selon l'une quelconque des revendications précédentes, **caractérisé en ce que** la disponibilité en oxygène dissous dans le fermenteur est assuré par le suivi du quotient respiratoire dès que la biomasse présente une teneur en lipides de plus de 25 %, de préférence plus de 30 % en poids sec de biomasse.

6. Procédé selon l'une quelconque des revendications précédentes, **caractérisé en ce que**, durant la période de suivi, le quotient respiratoire est maintenu à une valeur supérieure à 1,6, plus préférentiellement supérieure à 1,7 et plus préférentiellement encore supérieure à 1,8.

7. Procédé selon l'une quelconque des revendications précédentes, **caractérisé en ce que** le ratio métabolique Y O$_2$/S cumulé, correspondant au rapport entre la quantité d'O$_2$ consommé et la quantité de glucose consommé calculé depuis le début de la fermentation, est maintenu à une valeur inférieure à 0,28, préférentiellement inférieure à 0,27, plus préférentiellement inférieure à 0,26.

8. Procédé selon l'une quelconque des revendications précédentes, **caractérisé en ce que** le ratio métabolique Y O$_2$/S (correspondant au rapport entre la quantité d'O$_2$ consommé et la quantité de glucose consommé) observé durant la phase d'accumulation des lipides, de préférence lorsque la biomasse contient plus de 25 % de lipides, de préférence plus de 30 % de lipides (% exprimé en poids sec de biomasse), est maintenu à une valeur inférieure à 0,28, préférentiellement inférieure à 0,27, plus préférentiellement inférieure à 0,26

9. Procédé selon l'une quelconque des revendications précédentes, **caractérisé en ce que** la teneur en acide linoléique dans la biomasse produite est inférieure à 14% en poids par rapport au poids total des acides gras dans la biomasse sèche.

10. Procédé selon l'une quelconque des revendications précédentes, **caractérisé en ce que** le quotient respiratoire est suivi à l'aide d'un analyseur de gaz.

11. Procédé selon l'une quelconque des revendications précédentes, **caractérisé en ce que** la disponibilité en oxygène est contrôlée en faisant varier l'apport d'oxygène dans le milieu de fermentation par modulation de la vitesse d'agitation, de la contrepression ou de la concentration en oxygène dans l'air entrant.

**Patentansprüche**

1. Verfahren zur fermentativen Produktion von Biomasse von lipidreichen Mikroalgen der Gattung *Chlorella,* **dadurch gekennzeichnet, dass** die Mikroalgen unter heterotrophen Bedingungen kultiviert werden und dass das Verfahren wenigstens einen Schritt der Kultivierung umfasst, in dem die Biomasse Lipide akkumuliert und einen Lipidgehalt von mehr als 25 Trockengew.-% der Biomasse aufweist, in dem die Steuerung der Verfügbarkeit von gelöstem Sauerstoff durch die Kontrolle des respiratorischen Quotienten besagter Mikroalgen sichergestellt ist und in dem der respiratorische Quotient auf einen Wert über 1,5 gehalten wird, wobei der Linolsäuregehalt in der produzierten Biomasse unter 18 Gew.% bezogen auf das Fettsäuregesamtgewicht in der trockenen Biomasse beträgt.

2. Verfahren gemäß einem der vorhergehenden Ansprüche, **dadurch gekennzeichnet, dass** die Mikroalgen aus der Gruppe ausgewählt sind, bestehend aus *Chlorella vulgaris, Chlorella sorokiniana* und *Chlorella protothecoides.*

3. Verfahren gemäß einem der vorhergehenden Ansprüche, **dadurch gekennzeichnet, dass** die Mikroalgen *Chlorella protothecoides* sind.

4. Verfahren gemäß einem der vorhergehenden Ansprüche, **dadurch gekennzeichnet, dass** die mit besagtem Verfahren produzierte Biomasse einen Lipidgehalt von mehr als 30 Trockengew.-%, vorzugsweise mehr als 40 Trockengew.-% der Biomasse aufweist.

5. Verfahren gemäß einem der vorhergehenden Ansprüche, **dadurch gekennzeichnet, dass** die Verfügbarkeit von gelöstem Sauerstoff im Fermenter durch Kontrolle des respiratorischen Quotienten sichergestellt wird, sobald die Biomasse einen Lipidgehalt von mehr als 25 Trockengew.-%, vorzugsweise mehr als 30 Trockengew.-% der Biomasse aufweist.

6. Verfahren gemäß einem der vorhergehenden Ansprüche, **dadurch gekennzeichnet, dass**, während des Kontrollintervalls, der respiratorische Quotient auf einen Wert höher als 1,6, bevorzugter höher als 1,7 und noch bevorzugter höher als 1,8 gehalten wird.

7. Verfahren gemäß einem der vorhergehenden Ansprüche, **dadurch gekennzeichnet, dass** das kumulierte metabolische Verhältnis Y $O_2$/S, das dem seit Fermentationsbeginn berechneten Verhältnis von verbrauchter $O_2$-Menge zu verbrauchter Glucosemenge entspricht, auf einen Wert unter 0,28, bevorzugt unter 0,27, bevorzugter unter 0,26 gehalten wird.

8. Verfahren gemäß einem der vorhergehenden Ansprüche, **dadurch gekennzeichnet, dass** das metabolische Verhältnis Y $O_2$/S (entspricht dem Verhältnis von verbrauchter 02-Menge zu verbrauchter Glucosemenge), das während der Akkumulationsphase der Lipide festgestellt wird, bevorzugt, wenn die Biomasse mehr als 25 % Lipide, bevorzugt mehr als 30 % Lipide (ausgedrückt in Trockengew.-% der Biomasse) enthält, auf einen Wert unter 0,28, bevorzugt unter 0,27, bevorzugter unter 0,26 gehalten wird.

9. Verfahren gemäß einem der vorhergehenden Ansprüche, **dadurch gekennzeichnet, dass** der Linolsäuregehalt in der produzierten Biomasse weniger als 14 Gew-% bezogen auf den Fettsäuregesamtgehalt in der trockenen Biomasse beträgt.

10. Verfahren gemäß einem der vorhergehenden Ansprüche, **dadurch gekennzeichnet, dass** der respiratorische Quotient mithilfe eines Gasanalysators kontrolliert wird.

11. Verfahren gemäß einem der vorhergehenden Ansprüche, **dadurch gekennzeichnet, dass** die Sauerstoffverfügbarkeit durch Variieren der Sauerstoffzufuhr in das Fermentationsmedium durch Modulation der Rührgeschwindigkeit, des Gegendrucks oder der Sauerstoffkonzentration in der zugeführten Luft gesteuert wird.

**EP 3 036 316 B1**

**Claims**

1. A process for the fermentative production of biomass of lipid-rich microalgae of the *Chlorella* genus, **characterized in that** the microalgae are cultured under heterotrophic conditions and **in that** it comprises at least one culture step during which the biomass accumulates lipids and has a lipid content of more than 25% by dry weight of biomass, during which the control of dissolved oxygen availability in the fermenter is ensured by monitoring the respiratory quotient of said microalgae, and during which the respiratory quotient is maintained at a value greater than 1.5, the linoleic acid content in the biomass produced being less than 18% by weight relative to the total weight of fatty acids in the dry biomass.

2. The process according to any one of the preceding claims, **characterized in that** the microalgae are chosen from the group consisting of *Chlorella vulgaris, Chlorella sorokiniana* and *Chlorella protothecoides.*

3. The process according to any one of the preceding claims, **characterized in that** the microalgae are *Chlorella protothecoides.*

4. The process according to any one of the preceding claims, **characterized in that** the biomass produced by means of said process has a lipid content of more than 30% by dry weight of biomass, preferably of more than 40%.

5. The process according to any one of the preceding claims, **characterized in that** the dissolved oxygen availability in the fermenter is ensured by monitoring the respiratory quotient as soon as the biomass has a lipid content of more than 25%, preferably more than 30% by dry weight of biomass.

6. The process according to any one of the preceding claims, **characterized in that**, during the monitoring period, the respiratory quotient is maintained at a value greater than 1.6, preferably greater than 1.7, and more preferentially greater than 1.8.

7. The process according to any one of the preceding claims, **characterized in that** the cumulative Y $O_2$/S metabolic ratio, corresponding to the ratio between the amount of $O_2$ consumed and the amount of glucose consumed calculated from the beginning of the fermentation, is maintained at a value of less than 0.28, preferentially less than 0.27, more preferentially less than 0.26.

8. The process according to any one of the preceding claims, **characterized in that** the Y $O_2$/S metabolic ratio (corresponding to the ratio between the amount of $O_2$ consumed and the amount of glucose consumed) observed during the lipid accumulation phase, preferably when the biomass contains more than 25% of lipids, preferably more than 30% of lipids (% expressed by dry weight of biomass), is maintained at a value of less than 0.28, preferentially less than 0.27, more preferentially less than 0.26.

9. The process according to any one of the preceding claims, **characterized in that** the content of linoleic acid in the biomass produced is less than 14% by weight relative to the total weight of the fatty acids in the dry biomass.

10. The process according to any one of the preceding claims, **characterized in that** the respiratory quotient is monitored using a gas analyzer.

11. The process according to any one of the preceding claims, **characterized in that** the oxygen availability is controlled by varying the oxygen supply to the fermentation medium by modulating the stirring speed, the backpressure or the oxygen concentration in the entering air.

Figure 1

Figure 2

**Figure 3**

**Figure 4**

**Figure 5**

**RÉFÉRENCES CITÉES DANS LA DESCRIPTION**

**Documents brevets cités dans la description**

- WO 2010120923 A
- WO 2010045368 A
- WO 201012093 A
- FR 1356113